# EUROPÄISCHE PATENTSCHRIFT

(11) **EP 2 999 498 B1**
(45) Veröffentlichungstag und Bekanntmachung des Hinweises auf die Patenterteilung: **26.07.2017**
(21) Anmeldenummer: 14724114.5
(22) Anmeldetag: 16.05.2014
(51) Int. Cl.: A61M 1/36

(54) **VORRICHTUNG ZUR ÜBERWACHUNG EINES EXTRAKORPORALEN BLUTKREISLAUFS**
DEVICE FOR MONITORING AN EXTRACORPOREAL BLOOD CIRCUIT
DISPOSITIF DE SURVEILLANCE D'UN CIRCUIT SANGUIN EXTRACORPOREL

(30) Priorität: 23.05.2013 DE 102013008720
(43) Veröffentlichungstag der Anmeldung: 30.03.2016
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE); NÜRNBERGER, Thomas, 97705 Burkardroth (DE)
(74) Vertreter: Oppermann, Frank
(86) Internationale Anmeldenummer: PCT/EP2014/060171
(87) Internationale Veröffentlichungsnummer: WO 2014/187755

(56) Entgegenhaltungen:
- EP-A2- 0 995 451
- US-A- 6 077 443
- US-A1- 2012 271 161

## Beschreibung

Die Erfindung betrifft eine Vorrichtung zur Überwachung eines extrakorporalen Blutkreislaufs für eine extrakorporale Blutbehandlungsvorrichtung und eine extrakorporale Blutbehandlungsvorrichtung mit einer Vorrichtung zur Überwachung eines extrakorporalen Blutkreislaufs.

Auf dem Gebiet der Medizintechnik sind verschiedene Einrichtungen bekannt, mit denen über eine Schlauchleitung Patienten Flüssigkeiten entnommen oder Flüssigkeiten dem Patienten zugeführt werden könnten. Dabei erfolgt der Zugang zu dem Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicherzustellen. Daher ist es erforderlich, den Patientenzugang zu überwachten.

Einen ordnungsgemäßen Zugang zu dem Patienten setzen insbesondere die extrakorporalen Blutbehandlungsvorrichtungen voraus, die über einen extrakorporalen Blutkreislauf verfügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten beispielsweise über eine arterielle Schlauchleitung mit einer arteriellen Punktionskanüle Blut entnommen, das dem Patienten über eine venöse Schlauchleitung mit einer venösen Punktionskanüle wieder zugeführt wird. Die Überwachung des arteriellen und venösen Patientenzugangs kann auch beim Einsatz eines Doppellumenkatheters erfolgen, der insbesondere bei der akuten extrakorporalen Blutbehandlung eingesetzt wird.

Zur Überwachung des Patientenzugangs sind Überwachungsvorrichtungen bekannt, die auf den unterschiedlichsten Funktionsprinzipien beruhen. Sämtlichen Überwachungsvorrichtungen ist aber gemeinsam, dass überprüft wird, ob ein bestimmtes Kriterium vorliegt, das für einen nicht-ordnungsgemäßen Zustand des Gefäßzugangs als charakteristisch angesehen wird.

Aus der WO 2006/008866 A1 und der US 6,445,304 B1 sind Überwachungsvorrichtungen bekannt, die als charakteristisches Kriterium für einen ordnungsgemäßen Patientenzugang annehmen, dass die Haut des Patienten an der Punktionsstelle nicht feucht ist. Daher verfügen diese Überwachungsvorrichtungen über einen Feuchtigkeitssensor, der an der Punktionsstelle auf die Haut des Patienten aufgelegt wird. Bei einem nicht-ordnungsgemäßen Patientenzugang wird mit dem Feuchtigkeitssensor das Austreten von Blut an der Punktionsstelle zwar erkannt. Nachteilig ist aber, dass eine unbeabsichtigte Benetzung der Punktionsstelle mit Flüssigkeit zu einem Fehlalarm führen kann.

Andere Überwachungsvorrichtungen werten zur Überwachung eines Patientenzugangs charakteristische Größen aus, die in dem arteriellen und/oder venösen Zweig des extrakorporalen Blutkreislaufs gemessen werden. Es sind Überwachungsvorrichtungen bekannt, die als charakteristische Größe den Druck im arteriellen und/oder venösen Zweig überwachen. Bei einem Druckanstieg oder Druckabfall wird auf einen nicht-ordnungsgemäßen Gefäßzugang geschlossen. Darüber hinaus sind Überwachungsvorrichtungen bekannt, die charakteristische Druckpulse in dem arteriellen und/oder venösen Zweig des extrakorporalen Blutkreislaufs überwachen. Diese Druckpulse werden in dem extrakorporalen Blutkreislauf erzeugt oder können auf physiologische Ereignisse des Patienten zurückzuführen sein, der an den extrakorporalen Blutkreislauf angeschlossen ist. Beispielsweise ist es bekannt, Druckpulse im extrakorporalen Blutkreislauf zu überwachen, die im extrakorporalen Kreislauf durch die laufende Blutpumpe oder außerhalb des Blutkreislaufs durch den Herzschlag des Patienten erzeugt werden.

Überwachungsvorrichtungen, die eine Überwachung der Veränderung des Drucks oder von Druckpulsen im extrakorporalen Blutkreislauf vorsehen, sind beispielsweise in der WO 97/10013, WO 2009/127683 A1 und WO 2010/149726 A2 beschrieben.

In der EP 0 995 451 B1 wird pauschal vorgeschlagen, ein auf der Überwachung des arteriellen und venösen Drucks basierendes Verfahren zur Erhöhung der Sicherheit mit anderen Verfahren zur Erkennung eines fehlerhaften Gefäßzugangs zu kombinieren. Die WO 2009/127683 A1 schlägt vor, mehrere charakteristische Größen zu erfassen und für die Überwachung auf den Herzschlag des Patienten zurückzuführende Druckpulsen und von der laufenden Blutpumpe erzeugte Druckpulse heranzuziehen.

Der Erfindung liegt die Aufgabe zugrunde, einerseits die Sicherheit der extrakorporalen Blutbehandlung zu erhöhen und andererseits die Gefahr eines Fehlalarms zu verringern.

Die Lösung dieser Aufgabe erfolgt erfingdungsgemäß mit den Merkmalen von Anspruch 1. Die abhängigen Ansprüche betreffen bevorzugte Ausführungsformen der Erfindung.

Die erfindungsgemäße Vorrichtung geht davon aus, dass die Überwachung des arteriellen und/oder venösen Gefäßzugangs mit einem ersten und einem zweiten Verfahren erfolgt, bei denen jeweils das Vorliegen von mindestens einem Kriterium überprüft wird, das für einen nicht-ordnungsgemäßen Zustand des Gefäßzugangs charakteristisch ist, wobei sich die Kriterien des ersten und zweiten Verfahrens voneinander unterscheiden. Ein nicht-ordnungsgemäßer Patientenzugang wird auf der Grundlage der Überwachung mit dem ersten und zweiten Verfahren festgestellt, wobei ein nicht-ordnungsgemäßer Gefäßzugang festgestellt wird, wenn sowohl das mindestens eine Kriterium des ersten Verfahrens als auch das mindestens eine Kriterium des zweiten Verfahrens vorliegen. Aufgrund der erhöhten Redundanz wird die Zuverlässigkeit der Erkennung eines nicht-ordnungsgemäßen Gefäßzugangs verbessert. Die erfindungsgemäße Vorrichtung sieht vor, dass eine Unterbrechung der Blutbehandlung durch Schließen des venösen Verschlussorgans im venösen Zweig des extrakorporalen Blutkreislaufs nur dann erfolgen kann, wenn ein nicht-ordnungsgemäßer Gefäßzugang mit hoher Sicherheit erkannt worden ist. Ansonsten wird die Blutbehandlung nicht durch Schließen des venösen Verschlussorgans unterbrochen. Dies ist in der Praxis insofern von Vorteil, als das venöse Verschlussorgan der bekannten Blutbehandlungsvorrichtungen nach dem Schließen infolge eines Störfalls in einen definierten Verschlusszustand übergeht, der ein Öffnen des Verschlussorgans nur mit einem manuellen Eingriff von Seiten des medizinischen Personals nach der Vornahme der erforderlichen Maßnahmen erlauben soll.

Bei der erfindungsgemäßen Vorrichtung wird zunächst nur die Blutpumpe, die vorzugsweise eine okkludierende Blutpumpe ist, nach dem Feststellen des Vorliegens des mindestens einen Kriteriums des ersten Verfahrens gestoppt, während das venöse Verschlussorgan aber noch geöffnet bleibt. Nach dem Stoppen der Blutpumpe wird das Vorliegen des mindestens einen Kriteriums des zweiten Verfahrens überprüft. Das venöse Verschlussorgan wird erst dann geschlossen, wenn das mindestens eine Kriterium des zweiten Verfahrens vorliegt. Ansonsten wird die Blutpumpe zur Fortsetzung der Blutbehandlung wieder in Gang gesetzt. Da nach dem Starten der Blutpumpe das Verschlussorgan nicht geöffnet zu werden braucht, kann die Blutbehandlung ohne manuellen Eingriff fortgesetzt werden. Wenn die Punktionskanüle hingegen aus dem Gefäßzugang herausgerutscht sein sollte, d.h. ein Störfall tatsächlich vorliegen sollte, kann schon nach dem Stoppen der vorzugsweise okkludierenden Blutpumpe kein Blut mehr mittels der Pumpe aus der venösen Schlauchleitung gefördert werden, auch wenn das Verschlussorgan noch nicht geschlossen ist. Dadurch wird die Sicherheit der Blutbehandlung insgesamt erhöht und unnötige Unterbrechungen der Blutbehandlung werden vermieden.

In der vorliegenden Patentanmeldung wird unter dem arteriellen Zweig des extrakorporalen Blutkreislaufs mit dem arteriellen Patientenanschluss die Entnahmeleitung mit Entnahmenadel verstanden, mit denen Blut von dem Patienten entnommen wird und unter dem venösen Zweig des extrakorporalen Blutkreislaufs mit dem venösen Patientenanschluss wird die Rückgabeleitung mit Rückgabenadel verstanden, mit denen Blut zu dem Patienten zurückgeführt wird. Diese Definition der Begriffe "arterieller Zweig des extrakorporalen Blutkreislaufs mit dem arteriellen Patientenanschluss" und "venöser Zweig des extrakorporalen Blutkreislaufs mit dem venösen Patientenanschluss" betreffen daher gleichermaßen arterio-venöse und veno-venöse Gefäßzugänge.

Bei Blutbehandlungsvorrichtungen, die neben dem venösen Verschlussorgan auch über ein arterielles Verschlussorgan verfügen, bleiben nach dem Feststellen des mindestens einen Kriteriums der ersten Verfahrens das arterielle und venöse Verschlussorgan geöffnet, wobei das arterielle und venöse Verschlussorgan geschlossen werden, wenn das mindestens eine Kriterium des zweiten Verfahrens vorliegt.

Eine bevorzugte Ausführungsform sieht im Falle einer Störung einen akustischen und/oder optischen und/oder taktilen Alarm vor, der nach dem Feststellen des Vorliegens des mindestens einen Kriteriums des zweiten Verfahrens erzeugt wird.

Bei einer besonders bevorzugten Ausführungsform wird nach dem Feststellen des Vorliegens des mindestens einen Kriteriums des ersten Verfahrens ein erstes elektrisches Alarmsignal erzeugt, und nach dem Feststellen des Vorliegens des mindestens einen Kriteriums des zweiten Verfahrens ein zweites elektrisches Alarmsignal erzeugt. Das erste Alarmsignal kann einen Voralarm auslösen, beispielsweise einen optischen Alarm, insbesondere einen Hinweis auf dem Bedienfeld der Dialysemaschine oder eine Protokollierung, um das medizinische Personal auf eine mögliche Komplikation hinzuweisen. Das medizinische Personal wird mit dem Voralarm also frühzeitig auf eine mögliche Störung aufmerksam gemacht und kann spätestens nach dem zweiten Alarm die erforderlichen Maßnahmen treffen.

Für die Erfindung ist unerheblich, welche Kriterien dem ersten und zweiten Verfahren für die Überwachung des Blutkreislaufs zugrunde gelegt werden. Das erste und zweite Verfahren können beliebige Verfahren zur Überwachung des Gefäßzugangs sein. Die Verifizierung der Feststellung eines fehlerhaften Gefäßzugangs kann grundsätzlich nicht nur mit einem weiteren Verfahren, sondern auch mit mehreren Verfahren erfolgen. Es versteht sich von selbst, dass das Vorliegen eines Kriteriums für einen nicht-ordnungsgemäßen Gefäßzugang gleichbedeutend mit dem Nicht-Vorliegen eines Kriteriums für einen ordnungsgemäßen Gefäßzugang ist.

Das mindestens eine Kriterium des ersten und/oder zweiten Verfahrens kann die Veränderung einer charakteristischen Größe sein, die in dem arteriellen und/oder venösen Zweig des extrakorporalen Kreislaufs oder außerhalb des arteriellen und/oder venösen Zweiges des extrakorporalen Kreislaufs an der venösen und/oder arteriellen Punktionsstelle gemessen wird. In diesem Zusammenhang wird unter einer Veränderung einer Größe auch verstanden, dass die Größe nach dem Störfall nicht mehr vorliegt.

Die Veränderung der charakteristischen Größe kann die Veränderung des Drucks oder die Veränderung von Druckpulsen in dem arteriellen und/oder venösen Zweig des extrakorporalen Kreislaufs in Abhängigkeit von dem Zustand des arteriellen und/oder venösen Gefäßzugangs sein. Beispielsweise kann der Abfall des Blutdrucks bei einer kontinuierlichen Blutdruckmessung erfasst werden. Es können die von der laufenden Blutpumpe oder vom Herzschlag des Patienten erzeugten Druckpulse gemessen werden. Auch können durch kurzzeitiges Öffnen und/oder Schließen der Absperrorgane oder einer gezielten Veränderung der Förderarte der Blutpumpe oder der Ultrafiltrationsrate Druckpulse für die Überwachung des Blutkreislaufs erzeugt werden. Des Weiteren ist die Erzeugung von kurzzeitigen Temperaturschwankungen im Blutkreislauf möglich.

Als besonders vorteilhaft erweist sich die erfindungsgemäße Vorrichtung, wenn das zweite Verfahren zum Verifizieren des Vorliegens eines nicht-ordnungsgemäßen Gefäßzugangs auf einer Überwachung der Veränderung von im arteriellen und/oder venösen Zweig des extrakorporalen Kreislaufs gemessenen Druckpulsen beruht, die auf ein physiologisches Ereignis des an dem extrakorporalen Kreislauf angeschlossenen Patienten zurückzuführen sind, insbesondere durch den Herzschlag des Patienten erzeugt werden. Mit dem Stoppen der Blutpumpe bei geöffneten Absperrorganen werden die für die Messung der auf ein physiologisches Ereignis des Patienten zurückzuführenden Druckpulse optimale Voraussetzungen geschaffen, Da die Messung dieser Druckpulse nicht bei laufender Blutpumpe erfolgt, werden diese Druckpulse, die im Vergleich zu den von der Blutpumpe erzeugten Druckpulsen eine relativ kleine Amplitude haben, nicht von den Pulsen der Blutpumpe überlagert. Folglich ist es nicht erforderlich diese Druckpulse mit einer relativ aufwändigen Signalanalyse von den Pulsen der Blutpumpe zu trennen.

Bei einer besonders bevorzugten Ausführungsform werden die von dem Herzschlag des Patienten erzeugten Druckpulse im arteriellen und venösen Zweig des extrakorporalen Kreislaufs gemessen, wobei die Amplitude der im arteriellen Zweig gemessenen Druckpulse mit einem ersten Grenzwert und die Amplitude der im venösen Zweig gemessenen Druckpulse mit einem zweiten Grenzwert verglichen werden, wobei die Blutpumpe nur dann wieder in Gang gesetzt wird, wenn sowohl die Amplitude der im arteriellen Zweig gemessen Druckpulse größer als der erste Grenzwert als auch die Amplitude der im venösen Zweig gemessen Druckpulse größer als der zweite Grenzwert sind. Die Überwachung sowohl des arteriellen als auch venösen Zweigs des Blutkreislaufs stellt sicher, dass die Blutbehandlung nur dann fortgesetzt wird, wenn mit hoher Sicherheit ausgeschlossen ist, dass nicht doch ein Störfall vorliegt.

Eine weitere besonders bevorzugte Ausführungsform sieht ein Schließen des venösen und gegebenenfalls auch des arteriellen Absperrorgans schon dann vor, wenn nach dem Stoppen der Blutpumpe ein vorgegebenes Zeitintervall abgelaufen ist. Folglich muss die Feststellung, dass ein Störfall nicht vorliegt, so dass die Blutpumpe wieder gestartet werden kann, innerhalb eines Zeitfensters erfolgt sein.

Die erfindungsgemäße Vorrichtung zur Überwachung des extrakorporalen Blutkreislaufs verfügt über eine Steuer- und Recheneinheit, die derart konfiguriert ist, dass die für die Durchführung des erfindungsgemäßen Verfahrens erforderlichen Verfahrensschritte ausgeführt werden. Die Steuer- und Recheneinheit kann eine Datenverarbeitungseinheit, beispielsweise ein Mikroprozessor sein, auf der ein Datenverarbeitungsprogramm läuft.

Die Erfassung der für die Überwachung relevanten Größen kann mit den bekannten Messeinheiten erfolgen. Für die Messung des Drucks weist die Überwachungsvorrichtung beispielsweise eine Druck-Messeinheit auf.

Die erfindungsgemäße Überwachungsvorrichtung ist vorzugsweise Bestandteil der extrakorporalen Blutbehandlungsvorrichtung, so dass die Überwachungsvorrichtung von den in der Blutbehandlungsvorrichtung vorhandenen Komponenten Gebrauch machen kann. Die Steuer- und Recheneinheit der Überwachungsvorrichtung kann dann Bestandteil zentralen Steuer- und Recheneinheit der Blutbehandlungsvorrichtung sein. Die Steuerung der erforderlichen Komponenten erfolgt mit Steuersignalen, unter denen sämtliche Zeichen verstanden werden, mit denen Befehle oder Messwerte übertragen werden können.

Im Folgenden wird ein Ausführungsbeispiel der Erfindung unter Bezugnahme auf die einzige Figur näher erläutert, in der die wesentlichen Komponenten einer extrakorporalen Blutbehandlungsvorrichtung gezeigt sind, die über eine Vorrichtung zur Überwachung des extrakorporalen Blutkreislaufs verfügt.

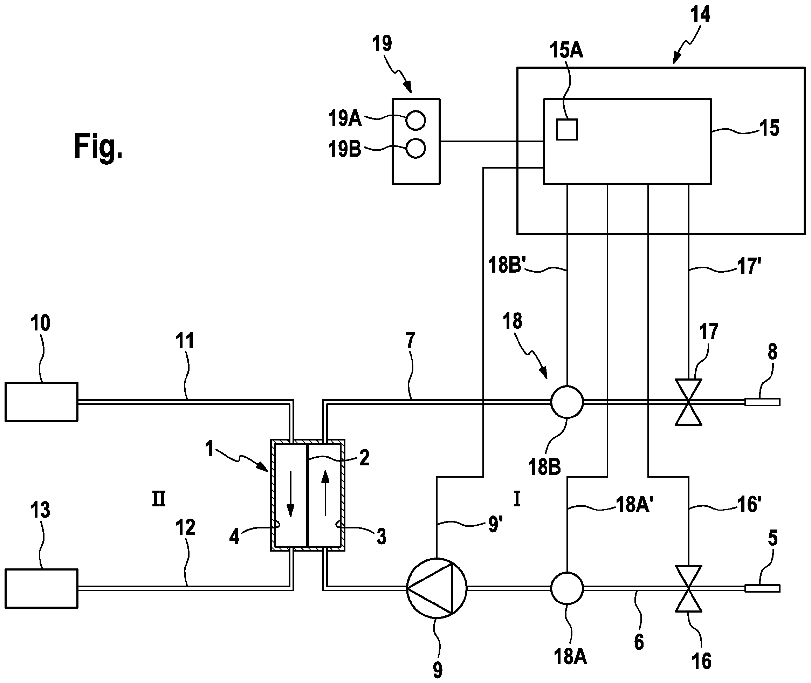

Fig. 1 zeigt die wesentlichen Komponenten einer extrakorporalen Blutbehandlungsvorrichtung, insbesondere Hämodialysevorrichtung, die über eine Vorrichtung zur Überwachung des Gefäßzugangs verfügt. Bei dem vorliegenden Ausführungsbeispiel ist die Überwachungsvorrichtung Bestandteil der Hämodialysevorrichtung.

Die Hämodialysevorrichtung weist als Blutbehandlungseinheit einen Dialysator oder Filter 1 auf, der durch eine semipermeable Membran 2 in eine Blutkammer 3 und eine Dialysierflüssigkeitskammer 4 unterteilt ist. An der nicht dargestellten Fistel oder dem Shunt des Patienten ist mittels einer arteriellen Punktionskanüle 5 als Patientenanschluss eine arterielle Schlauchleitung 6 angeschlossen, die zu einem Einlass der Blutkammer 3 des Dialysators 1 führt. Von dem Auslass der Blutkammer 3 des Dialysators 1 geht eine venöse Schlauchleitung 7 ab, die mittels einer venösen Punktionskanüle 8 als Patientenanschluss an der Fistel oder dem Shunt des Patienten angeschlossen ist. In die arterielle Schlauchleitung 6 ist eine Blutpumpe 9 geschaltet, die das Blut im extrakorporalen Blutkreislauf I fördert. Die Blutpumpe 9 ist vorzugsweise eine okkludierende Schlauchpumpe. Die arterielle und venöse Schlauchleitung bilden den arteriellen oder venösen Zweig 6, 7 des extrakorporalen Blutkreislaufs.

Der Dialysierflüssigkeitskreislauf II der Dialysevorrichtung umfasst eine Dialysierflüssigkeitsquelle 10, an der eine Dialysieiflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine nicht dargestellte Dialysierflüssigkeitspumpe geschaltet.

Die Steuerung der Dialysevorrichtung erfolgt mit einer zentralen Steuer- und Recheneinheit 14, die einen Mikroprozessor aufweist, der derart programmiert ist, dass die für die Steuerung der einzelnen Komponenten sowie der Erfassung und Auswertung von Messwerten erforderlichen Schritte durchgeführt werden. Die Steuer- und Recheneinheit 15 der Überwachungsvorrichtung ist bei dem vorliegenden Ausführungsbeispiel Bestandteil der zentralen Steuer- und Recheneinheit 14 der Dialysevorrichtung.

An der arteriellen Schlauchleitung 6 ist stromab der arteriellen Kanüle 5 und stromauf der Blutpumpe 9 ein arterielles Absperrorgan 16 und an der venösen Schlauchleitung 7 ist stromauf der venösen Kanüle 8 ein venöses Absperrorgan 17 vorgesehen. Die Absperrorgane 16, 17 können elektromagnetisch betätigbare Schlauchklemmen sein. Das arterielle Absperrorgan 16 kann grundsätzlich aber auch entfallen.

Des Weiteren weist die Überwachungsvorrichtung eine Druck-Messeinheit 18 auf, die über einen arteriellen Drucksensor 18A und einen venösen Drucksensor 18B aufweist, die zum Messen des Drucks in der arteriellen bzw. venösen Schlauchleitung 6, 7 konfiguriert sind.

Darüber hinaus verfügt die Überwachungsvorrichtung über eine Alarmeinheit 19, die bei dem vorliegenden Ausführungsbeispiel Bestandteil der Alarmeinheit der Blutbehandlungsvorrichtung ist. Die Alarmeinheit 19 weist einen ersten Signalgeber 19A und einen zweiten Signalgeber 19B auf. Der erste Signalgeber 19A gibt nur einen Voralarm, beispielsweise nur ein optisches Signal, einen Hinweis auf dem Bildschirm der Maschine oder eine entsprechende Protokollierung, während der zweite Signalgeber 19B einen akustischen und/oder optischen und/oder taktilen Alarm gibt, der sofort wahrgenommen werden kann.

Zur Steuerung der einzelnen Komponenten und zur Erfassung der Messwerte sind die Blutpumpe 9 mit einer Steuerleitung 9', die Alarmeinheit 19 mit einer Steuerleitung 19', das arterielle und venöse Absperrorgan 16, 17 mit Steuerleitungen 16', 17' und der arterielle und venöse Drucksensor 18A, 18B mit Steuerleitungen 18A', 18B' mit der zentralen Steuer- und Recheneinheit 15 verbunden.

Die Steuer- und Recheneinheit 15 ist derart programmiert, dass während der Blutbehandlung fortlaufend der arterielle und venöse Druck mit den Drucksensoren 18A, 18B gemessen wird. Für die Überwachung des Gefäßzugangs, insbesondere des venösen Gefäßzugangs, werden aus den Druckmesswerten charakteristische Werte berechnet, die mit vorgegebenen Grenzwerten verglichen werden. Die Steuer- und Recheneinheit 15 schließt auf einen möglichen fehlerhaften Gefäßzugang, wenn die Summe und/oder Differenz der arteriellen und venösen Druckmesswerte außerhalb vorgegebener Grenzwertfenster liegen. Dieses Verfahren, das in der WO 2008/006559 A1 im Einzelnen beschrieben ist, soll aber nur als ein Beispiel für ein Überwachungsverfahren dienen. Alternativ können auch die von der Blutpumpe 9 erzeugten Drucksignale ausgewertet werden. Es ist aber auch möglich, den Gefäßzugang mit einem Feuchtigkeitssensor zu überwachen. Eine Überwachungsvorrichtung mit einem derartigen Feuchtigkeitssensor ist beispielsweise aus der WO 2011/116943 bekannt.

Wenn mit dem oben beschriebenen Verfahren das Vorliegen eines fehlerhaften Gefäßzugangs festgestellt wird, d.h. die Summe und/oder Differenz der arteriellen und venösen Druckmesswerte außerhalb vorgegebener Grenzwertfenster liegen, erzeugt die Steuer- und Recheneinheit 15 ein Steuersignal für die Blutpumpe, so dass die Blutpumpe 9 gestoppt wird. Die arterielle und venöse Schlauchklemme 16, 17 bleiben aber geöffnet. Die Steuer- und Recheneinheit 15 erzeugt weiterhin ein Steuersignal für die Alarmeinheit 19, so dass der erste Signalgeber 19A einen Voralarm gibt. Darüber hinaus wird ein Zeitglied 15A der Steuer- und Recheneinheit 15 in Gang gesetzt.

Die okkludierende Blutpumpe 9 trennt somit den arteriellen Zweig 6 des extrakorporalen Blutkreislaufs von dem arteriellen Gefäßzugang ab. Dadurch wird verhindert, dass Blut aus der venösen Punktionskanüle 8 gepumpt werden kann, wenn die venöse Kanüle aus dem venösen Gefäßzugang herausgerutscht sein sollte. Dieser Störfall wird nunmehr mit einem zweiten Überwachungsverfahren, das sich in den Überwachungskriterien von dem ersten Verfahren unterscheidet verifiziert.

Bei dem vorliegenden Ausführungsbeispiel ist das zweite Überwachungsverfahren ein Verfahren zur Überwachung von Druckpulsen, die vom Herzschlag des Patienten erzeugt werden, der an die arterielle und venöse Schlauchleitung 6, 7 angeschlossen ist. Diese vom Herzen erzeugten Druckpulse können sich bei noch offenen Schlauchklemmen 16, 17 bis in das Schlauchleitungssystem ausbreiten, so dass sie von dem arteriellen und venösen Drucksensor 18A, 18B erfasst werden. Da die Blutpumpe 9 still steht, werden von der Blutpumpe keine Druckpulse erzeugt. Daher werden von dem arteriellen und venösen Drucksensor im Wesentlichen nur die Druckpulse erfasst, die vom Herzen erzeugt werden. Zumindest sind diese Druckpulse nicht von Druckpulsen der Blutpumpe 9 überlagert. In der Steuer- und Recheneinheit 15 werden die Druckpulse des Herzens von Störsignalen befreit und ausgewertet, um nach den bekannten Verfahren feststellen zu können, ob ein fehlerhafter Gefäßzugang vorliegt. Die Detektion der Druckpulse des Herzens kann auch eine spektrale Analyse der Druckschwankungen einschließen. Ein derartiges Überwachungsverfahren ist beispielsweise in der WO 97/10013 beschrieben.

Für die Feststellung eines fehlerhaften Gefäßzugangs reicht die Auswertung der arteriellen Druckpulse im arteriellen Zweig oder der venösen Druckpulse im venösen Zweig des extrakorporalen Kreislaufs grundsätzlich aus. Vorzugsweise werden die venösen Druckpulse überwacht, um einen fehlerhaften Gefäßzugang auf der venösen Seite feststellen zu können. Ein Ausführungsbeispiel sieht die Auswertung sowohl der arteriellen als auch venösen Druckpulse des Herzens vor.

Bei einem Ausführungsbeispiel ist die Steuer- und Recheneinheit 15 derart ausgebildet, dass die Amplitude der im arteriellen Zweig gemessenen Druckpulse mit einem ersten Grenzwert und die Amplitude der im venösen Zweig gemessenen Druckpulse mit einem zweiten Grenzwert verglichen werden, wobei die Blutpumpe wieder in Gang gesetzt wird, wenn die Amplitude der im arteriellen Zweig gemessenen Druckpulse größer als der erste Grenzwert und die Amplitude der im venösen Zweig gemessenen Druckpulse größer als der zweite Grenzwert sind. Ein alternatives Kriterium für das Starten der Blutpumpe kann aber auch die Messung nur venöser Druckpulse sein.

Eine besonders bevorzugte Ausführungsform sieht eine Überwachung der Frequenz sowohl der arteriellen als auch venösen Druckpulse vor. Bei dieser Ausführungsform ist die Steuer- und Recheneinheit 15 derart ausgebildet, dass die Frequenz der arteriellen Druckpulse mit der Frequenz der venösen Druckpulse verglichen wird. Wenn die Differenz der Frequenzen größer als ein vorgegebener Grenzwert ist, wird auf einen nicht-ordnungsgemäßen Zustand geschlossen, obwohl sowohl arterielle als auch venöse Drucksignale detektiert werden.

Wenn im arteriellen und venösen Zweig des extrakorporalen Blutkreislaufs Druckpulse des Herzens detektiert werden, schließt die Steuer- und Recheneinheit 15 darauf, dass ein Störfall nicht vorliegt. In diesem Fall wird von der Steuer- und Recheneinheit 15 ein Steuersignal erzeugt, so dass die Blutpumpe 9 zur Fortsetzung der Blutbehandlung wieder in Gang gesetzt wird. Folglich ist die Blutbehandlung nur kurzzeitig unterbrochen gewesen, und die Fortsetzung der Blutbehandlung erfolgt automatisch ohne Eingriff des medizinischen Personals.

Wenn im venösen Zweig des extrakorporalen Blutkreislaufs Druckpulse des Herzens detektiert und im arteriellen Zweig des extrakorporalen Kreislaufs Druckpulse nicht detektiert werden, schließt die Steuer- und Recheneinheit 15 auch darauf, dass ein Störfall nicht zwingend vorliegt. In diesem Fall wird von der Steuer- und Recheneinheit 15 ein Steuersignal erzeugt, so dass die Blutpumpe 9 zur Fortsetzung der Blutbehandlung wieder in Gang gesetzt wird. Folglich ist die Blutbehandlung nur kurzzeitig unterbrochen gewesen, und die Fortsetzung der Blutbehandlung erfolgt wieder automatisch ohne Eingriff des medizinischen Personals. In diesem Fall kann gegebenenfalls ein Voralarm gegeben werden.

Wenn hingegen in der venösen Schlauchleitung 7 Druckpulse des Herzens nicht detektiert werden, so wird darauf geschlossen, dass ein nicht-ordnungsgemäßer Gefäßzugang tatsächlich vorliegt, so dass die Blutbehandlung unterbrochen wird.

Auf einen Störfall wird auch dann geschlossen, wenn in der arteriellen und der venösen Schlauchleitung 16, 17 keine Druckpulse des Herzens detektiert werden. Dieser Fall dürfte in der Praxis aber kaum auftreten, da hierzu beide Kanülen gleichzeitig diskonnektiert sein müssten.

Wenn auf einen Störfall geschlossen wird, erzeugt die Steuer- und Recheneinheit 15 ein Steuersignal für das arterielle und venöse Absperrorgan 16, 17, so dass die Absperrorgane geschlossen werden. Damit sind die arterielle und venöse Leitung 6, 7 vollständig gegenüber dem Patienten verschlossen. Die Steuer- und Recheneinheit 15 erzeugt weiterhin ein Steuersignal für die Alarmeinheit 19, so dass der zweite Signalgeber 19B einen vorzugsweise akustischen Alarm gibt. Nach dem akustischen Alarm können dann vom medizinischen Personal die erforderlichen Maßnahmen getroffen werden.

Während der Verifizierung des zuvor erkannten Störfalls wird fortlaufend von der Steuer- und Recheneinheit 15 überwacht, ob ein bestimmtes Zeitintervall, das von dem Zeitglied vorgegeben ist, noch nicht abgelaufen ist. Nach Ablauf des Zeitintervalls werden die arterielle und venöse Schlauchklemme 16, 17 aus Sicherheitsgründen automatisch geschlossen. Dadurch wird sichergestellt, dass eine Verifizierung des Störfalls und eine Fortsetzung der Blutbehandlung nur innerhalb zeitlich enger Grenzen erfolgen können.

## Patentansprüche

1. Vorrichtung zur Überwachung eines extrakorporalen Blutkreislaufs für eine Vorrichtung zur extrakorporalen Blutbehandlung,
wobei die extrakorporale Blutbehandlungsvorrichtung
einen extrakorporalen Blutkreislauf (I) mit einem zu einer Blutbehandlungseinheit (1) führenden arteriellen Zweig (6) mit einem arteriellen Patientenanschluss (5), und mit einem von der Blutbehandlungseinheit abgehenden venösen Zweig (7), in dem stromauf eines venösen Patientenanschlusses (8) ein venöses Verschlussorgan (17) vorgesehen ist, und eine Blutpumpe (9) zum Fördern von Blut im extrakorporalen Kreislauf aufweist,
wobei die Überwachungsvorrichtung eine Steuer- und Recheneinheit (15) aufweist, die derart konfiguriert ist,
dass ein erstes und ein zweites Verfahren zum Überwachen des arteriellen und/oder venösen Gefäßzugangs durchführbar sind, bei denen jeweils das Vorliegen von mindestens einem Kriterium überprüft wird, das für einen nicht-ordnungsgemäßen Zustand des Gefäßzugangs charakteristisch ist, wobei sich das mindestens eine Kriterium des ersten Verfahrens und das mindestens eine Kriterium des zweiten Verfahren voneinander unterscheiden, und
dass ein nicht-ordnungsgemäßer Gefäßzugangs auf der Grundlage der Überwachung mit dem ersten und zweiten Verfahren feststellbar ist,
**dadurch gekennzeichnet, dass**
die Steuer- und Recheneinheit (15) derart konfiguriert ist, dass nach dem Feststellen des Vorliegens des mindestens einen Kriteriums des ersten Verfahrens ein Steuersignal zum Stoppen der Blutpumpe (9) erzeugt wird, während das venöse Verschlussorgan (16, 17) geöffnet bleibt, und
nach der Erzeugung des Steuersignals zum Stoppen der Blutpumpe (9) das Vorliegen des mindestens einen Kriteriums mit dem zweiten Verfahrens überprüft wird,
wobei ein Steuersignal zum Schließen des venösen Verschlussorgans (17) erzeugt wird, wenn das mindestens eine Kriterium des zweiten Verfahrens vorliegt, und ein Steuersignal zum Starten der Blutpumpe (9) erzeugt wird, wenn das mindestens eine Kriterium des zweiten Verfahrens nicht vorliegt.

2. Vorrichtung nach Anspruch 1, **dadurch gekennzeichnet, dass** in dem arteriellen Zweig (6) stromab des arteriellen Patientenanschlusses (5) ein arterielles Verschlussorgan (16) vorgesehen ist, wobei die Steuer- und Recheneinheit (15) derart konfiguriert ist, dass nach dem Feststellen des Vorliegens des mindestens einen Kriteriums des ersten Verfahrens das arterielle und venöse Verschlussorgan (16, 17) geöffnet bleiben, und nach dem Stoppen der Blutpumpe (9) das Vorliegen des mindestens einen Kriteriums nach dem zweiten Verfahrens überprüft wird, wobei das arterielle und venöse Verschlussorgan (16, 17) geschlossen werden, wenn das mindestens eine Kriterium des zweiten Verfahrens vorliegt, und die Blutpumpe (9) wieder in Gang gesetzt wird, wenn das mindestens eine Kriterium des zweiten Verfahrens nicht vorliegt.

3. Vorrichtung nach Anspruch 1 oder 2, **dadurch gekennzeichnet, dass** die Überwachungsvorrichtung eine Alarmeinheit (19) aufweist und die Steuer- und Recheneinheit (15) derart konfiguriert ist, dass nach dem Feststellen des Vorliegens des mindestens einen Kriteriums des zweiten Verfahrens Alarm gegeben wird.

4. Vorrichtung nach einem der Ansprüche 1 bis 3, **dadurch gekennzeichnet, dass** die Überwachungsvorrichtung eine Alarmeinheit (19) mit einem ersten und zweiten Alarmgeber (19A, 19B) aufweist, und die Steuer- und Recheneinheit(15) derart konfiguriert ist, dass nach dem Feststellen des Vorliegens des mindestens einen Kriteriums des ersten Verfahrens ein erstes Alarmsignal erzeugt wird, und nach dem Feststellen des Vorliegens des mindestens einen Kriteriums des zweiten Verfahrens ein zweites Alarmsignal erzeugt wird.

5. Vorrichtung nach einem der Ansprüche 1 bis 4, **dadurch gekennzeichnet, dass** das mindestens eine Kriterium des ersten und/oder zweiten Verfahrens die Veränderung einer charakteristischen Größe ist, die in dem arteriellen und/oder venösen Zweig (6, 7) des extrakorporalen Kreislaufs (I) gemessen wird, oder die Veränderung einer charakteristischen Größe ist, die außerhalb des arteriellen und/oder venösen Zweigs des extrakorporalen Kreislaufs an der venösen und/oder arteriellen Punktionsstelle gemessen wird.

6. Vorrichtung nach Anspruch 5, **dadurch gekennzeichnet, dass** die Veränderung der charakteristischen Größe die Veränderung des Drucks oder die Veränderung von Druckpulsen in dem arteriellen und/oder venösen Zweig (6, 7) des extrakorporalen Kreislaufs (I) in Abhängigkeit von dem Zustand des arteriellen und/oder venösen Gefäßzugang ist.

7. Vorrichtung nach Anspruch 5 oder 6, **dadurch gekennzeichnet, dass** das mindestens eine Kriterium des zweiten Verfahrens die Veränderung von im arteriellen und/oder venösen Zweig (6, 7) des extrakorporalen Kreislaufs (I) gemessenen Druckpulsen ist, die auf ein physiologisches Ereignis des an dem extrakorporalen Kreislauf angeschlossenen Patienten, insbesondere des Herzschlags des Patienten zurückzuführen sind.

8. Vorrichtung nach Anspruch 7, dadurch gekenntzeichnet, dass die Überwachungsvorrichtung eine Druck-Messeinheit (18) aufweist, und die Steuer- und Recheneinheit (15) derart konfiguriert ist, dass die von dem Herzschlag des Patienten erzeugten Druckpulse im arteriellen und venösen Zweig (6, 7) des extrakorporalen Kreislaufs (I) gemessen werden, wobei die Amplitude der im arteriellen Zweig gemessenen Druckpulse mit einem ersten Grenzwert und die Amplitude der im venösen Zweig gemessenen Druckpulse mit einem zweiten Grenzwert verglichen werden,
und dass die Steuer- und Recheneinheit (15) derart konfiguriert ist, dass das Steuersignal zum Starten der Blutpumpe (9) erzeugt wird, wenn die Amplitude der im arteriellen Zweig (6) gemessenen Druckpulse größer als der erste Grenzwert und die Amplitude der im venösen Zweig (7) gemessenen Druckpulse größer als der zweite Grenzwert sind.

9. Vorrichtung nach einem der Ansprüche 1 bis 8, **dadurch gekennzeichnet, dass** die Überwachungsvorrichtung ein Zeitglied (15A) aufweist, und die Steuer- und Recheneinheit (15) derart konfiguriert ist, dass nach dem Stoppen der Blutpumpe (9) das Zeitglied (15A) in Gang gesetzt wird und ein Steuersignal zum Schließen des venösen Verschlussorgans (17) erzeugt wird, wenn das Zeitintervall abgelaufen ist.

10. Vorrichtung nach einem der Ansprüche 1 bis 9, **dadurch gekennzeichnet, dass** die Blutpumpe (9) eine okkludierende Blutpumpe ist.

11. Vorrichtung zur extrakorporalen Blutbehandlung, die einen extrakorporalen Blutkreislauf (I) mit einem zu einer Blutbehandlungseinheit (1) führenden arteriellen Zweig (6) mit einem arteriellen Patientenanschluss (5), und mit einem von der Blutbehandlungseinheit (19) abgehenden venösen Zweig (7), in dem stromauf eines venösen Patientenanschlusses (8) ein venöses Verschlussorgan (17) vorgesehen ist, und eine Blutpumpe (9) zum Fördern von Blut im extrakorporalen Kreislauf (I) und eine zentrale Steuer und Recheneinheit (15) zum Steuern der Blutpumpe (9) und des venösen Absperrorgans (17) aufweist,
**dadurch gekennzeichnet, dass** die Blutbehandlungsvorrichtung eine Vorrichtung zur Überwachung des extrakorporalen Blutkreislaufs nach einem der Ansprüche 1 bis 10 aufweist, wobei die zentrale Steuer- und Recheneinheit (15) zum Steuern der Blutpumpe (9) und des venösen Absperrorgans (17) derart konfiguriert ist, dass nach dem Empfang des Steuersignals der Überwachungsvorrichtung zum Stoppen der Blutpumpe (9) die Blutpumpe gestoppt und nach dem Empfang des Steuersignals der Überwachungsvorrichtung zum Starten der Blutpumpe die Blutpumpe gestartet und nach dem Empfang des Steuersignal zum Schließen des venösen Verschlussorgans das venöse Verschlussorgan (17) geschlossen wird.

## Claims

1. Device for monitoring an extracorporeal blood flow for a device for extracorporeal blood treatment,
the extracorporeal blood treatment device
having an extracorporeal blood flow (I) with an arterial branch (6) that leads to a blood treatment unit (1) and that has an arterial patient connection (5), and with a venous branch (7) that goes from the blood treatment unit and in which a venous cut-off unit (17) is provided upstream of a venous patient connection (8), and having a blood pump (9) for conveying blood in the extracorporeal flow,
the monitoring device having a control and computing unit (15) that is configured such that
a first method and a second method for monitoring the arterial and/or venous access may be performed, in each of which the presence of at least one criterion is checked that is characteristic of a vascular access condition that is out of proper order, the at least one criterion for the first method and the at least one criterion for the second method being distinguished from one another, and,
such that a vascular access that is not in proper order may be established based on the monitoring with the first and second methods,
**characterized in that**
the control and computing unit (15) is configured such that a control signal is generated for stopping the blood pump (9) once the presence of the at least one criterion for the first method has been established , while the venous cut-off unit (16, 17) remains open, and
the presence of the at least one criterion is checked with the second method once the control signal for stopping the blood pump (9) has been generated,
a control signal for closing the venous cut-off unit (17) being generated if the at least one criterion for the second method is present, and a control signal for starting the blood pump (9) being generated if the at least one criterion for the second method is not present.

2. Device in accordance with claim 1, **characterized in that** an arterial cut-off unit (16) is provided in the arterial branch (6) downstream of the arterial patient connection (5), the control and computing unit (15) being configured such that once the presence of the at least one criterion for the first method has been established the arterial and venous cut-off units (16, 17) remain open, and there is a check for the presence of the at least one criterion according to the second method once the blood pump (9) has been stopped, the arterial and venous cut-off units (16, 17) being closed if the at least one criterion for the second method is present, and the blood pump (9) being restarted if the at least one criterion for the second method is not present.

3. Device in accordance with claim 1 or 2, **characterized in that** the monitoring device has an alarm unit (19) and the control and computing device (15) is configured such that an alarm is provided once the presence of the at least one criterion for the second method has been established.

4. Device in accordance with any of claims 1 to 3, **characterized in that** the monitoring device has an alarm unit (19) with a first and a second alarm generator (19A, 19B), and the control and computing unit (15) is configured such that a first alarm signal is generated once the presence of the at least one criterion for the first method has been established, and a second alarm signal is generated once the presence of the at least one criterion for the second method has been established.

5. Device in accordance with any of claims 1 to 4, **characterized in that** the at least one criterion for the first and/or second method is the change in a characteristic variable that is measured in the arterial and/or venous branch (6, 7) of the extracorporeal flow (I), or is the change in a characteristic variable that is measured outside of the arterial and/or venous branch of the extracorporeal flow at the venous and/or arterial puncture site.

6. Device in accordance with claim 5, **characterized in that** the change of the characteristic variable is the change in the pressure or the change in the pressure pulses in the arterial and/or venous branch (6, 7) of the extracorporeal flow (I) as a function of the condition of the arterial and/or venous access.

7. Device in accordance with claim 5 or 6, **characterized in that** the at least one criterion for the second method is the change in the pressure pulses measured in the arterial and/or venous branch (6, 7) of the extracorporeal flow (I) that derives from a physiological event in the patient connected to the extracorporeal flow, especially the heartbeat of the patient.

8. Device in accordance with claim 7, **characterized in that** the monitoring device has a pressure measuring unit (18), and the control and computing device (15) is configured such that the pressure pulses produced by the heartbeat of the patient are measured in the arterial and venous branches (6, 7) of the extracorporeal flow (I), the amplitude of the pressure pulses measured in the arterial branch being compared to a first limit and the amplitude of the pressure pulses measured in the venous branch being compared to a second limit,
and **in that** the control and computing device (15) is configured such that the control signal for starting the blood pump (9) is generated when the amplitude of the pressure pulses measured in the arterial branch (6) is greater than the first limit and the amplitude of the pressure pulses measured in the venous branch (7) is greater than the second limit.

9. Device in accordance with any of claims 1 to 8, **characterized in that** the monitoring device has a timing element (15A), and the control and computing unit (15) is configured such that once the blood pump (9) has been stopped, the timing element (15A) is started and a control signal for closing the venous cut-off unit (17) is generated when the time interval has elapsed.

10. Device in accordance with any of claims 1 to 9, **characterized in that** the blood pump (9) is an occlusion pump.

11. Device for extracorporeal blood treatment, which device has an extracorporeal blood flow (I) with an arterial branch (6) that leads to a blood treatment unit (1) and that has an arterial patient connection (5), and with a venous branch (7) that goes out of the blood treatment unit (19) and in which a venous cut-off unit (17) is provided upstream of a venous patient connection (8), and has a blood pump (9) for conveying blood in the extracorporeal flow (I) and a central control and computing unit (15) for controlling the blood pump (9) and the venous cut-off unit (17),
**characterized in that** the blood treatment device has a device for monitoring the extracorporeal blood flow in accordance with any of claims 1 to 10, the central control and computing unit (15) being configured for controlling the blood pump (9) and the venous cut-off unit (17) such that the blood pump is stopped after the receipt of the control signal from the monitoring device for stopping the blood pump (9), and the blood pump is started after the receipt of the control signal from the monitoring device for starting the blood pump, and the venous cut-off unit (17) is closed after the receipt of the control signal for closing the venous cut-off unit.

## Revendications

1. Dispositif servant à surveiller un circuit sanguin extracorporel pour un dispositif de traitement du sang extracorporel,
dans lequel le dispositif de traitement du sang extracorporel
présente un circuit sanguin extracorporel (I) avec un embranchement artériel (6) menant à une unité de traitement du sang (1) avec un raccordement artériel à un patient (5), et avec un embranchement veineux (7) partant de l'unité de traitement du sang, dans lequel un organe de fermeture veineux (17) est prévu en amont d'un raccordement veineux au patient (8), et une pompe à sang (9) servant à refouler le sang dans le circuit extracorporel, dans lequel le dispositif de surveillance présente une unité de commande et de calcul (15), qui est configurée de telle manière
qu'un premier et un deuxième procédé servant à surveiller l'accès vasculaire artériel et/ou veineux peuvent être mis en oeuvre, dans lesquels respectivement la présence d'au moins un critère est vérifiée, lequel est caractéristique d'un état non conforme de l'accès vasculaire, dans lequel l'au moins un critère du premier procédé et l'au moins un critère du deuxième procédé diffèrent l'un de l'autre, et
qu'un accès vasculaire non conforme peut être constaté sur la base de la surveillance avec le premier et le deuxième procédé,
**caractérisé en ce que**
l'unité de commande et de calcul (15) est configurée de telle manière qu'une fois la présence de l'au moins un critère du premier procédé constatée, un signal de commande est généré pour stopper la pompe à sang (9), tandis que l'organe de fermeture veineux (16, 17) reste ouvert, et
une fois le signal de commande servant à stopper la pompe à sang (9) généré, la présence de l'au moins un critère est vérifiée avec le deuxième procédé,
dans lequel un signal de commande est généré afin de fermer l'organe de fermeture veineux (17) quand l'au moins un critère du deuxième procédé est présent, et un signal de commande est généré afin de démarrer la pompe à sang (9) quand l'au moins un critère du deuxième procédé n'est pas présent.

2. Dispositif selon la revendication 1, **caractérisé en ce qu'**un organe de fermeture artériel (16) est prévu dans l'embranchement artériel (6) en aval du raccordement artériel au patient (5), dans lequel l'unité de commande et de calcul (15) est configurée de telle manière qu'une fois la présence de l'au moins un critère du premier procédé constatée, l'organe de fermeture artériel et l'organe de fermeture veineux (16, 17) restent ouverts, et qu'une fois la pompe à sang (9) stoppée, la présence de l'au moins un critère est vérifiée selon le deuxième procédé, dans lequel l'organe de fermeture artériel et l'organe de fermeture veineux (16, 17) sont fermés, quand l'au moins un critère du deuxième procédé est présent, et la pompe à sang (9) est remise en marche quand l'au moins un critère du deuxième procédé n'est pas présent.

3. Dispositif selon la revendication 1 ou 2, **caractérisé en ce que** le dispositif de surveillance présente une unité d'alarme (19), et l'unité de commande et de calcul (15) est configurée de telle manière qu'une fois la présence de l'au moins un critère du deuxième procédé constatée, l'alarme est donnée.

4. Dispositif selon l'une quelconque des revendications 1 à 3, **caractérisé en ce que** le dispositif de surveillance présente une unité d'alarme (19) avec un premier et un deuxième émetteur d'alarme (19A, 19B), et l'unité de commande et de calcul (15) est configurée de telle manière qu'une fois la présence de l'au moins un critère du premier procédé constatée, un premier signal d'alarme est généré, et qu'une fois la présence de l'au moins un critère du deuxième procédé constatée, un deuxième signal d'alarme est généré.

5. Dispositif selon l'une quelconque des revendications 1 à 4, **caractérisé en ce que** l'au moins un critère du premier et/ou du deuxième procédé est la variation d'une grandeur caractéristique, qui est mesurée dans l'embranchement artériel et/ou veineux (6, 7) du circuit extracorporel (I), ou est la variation d'une grandeur caractéristique, qui est mesurée à l'extérieur de l'embranchement artériel et/ou veineux du circuit extracorporel au niveau du point de ponction veineux et/ou artériel.

6. Dispositif selon la revendication 5, **caractérisé en ce que** la variation de la grandeur caractéristique est la variation de la pression ou la variation d'impulsions de pression dans l'embranchement artériel et/ou veineux (6, 7) du circuit extracorporel (I) en fonction de l'état de l'accès vasculaire artériel et/ou veineux.

7. Dispositif selon la revendication 5 ou 6, **caractérisé en ce que** l'au moins un critère du deuxième procédé est la variation d'impulsions de pression mesurées dans l'embranchement artériel et/ou veineux (6, 7) du circuit extracorporel (I), qui sont à relier à un événement physiologique du patient raccordé au circuit extracorporel, en particulier au rythme cardiaque du patient.

8. Dispositif selon la revendication 7, **caractérisé en ce que** le dispositif de surveillance présente une unité de mesure de pression (18), et l'unité de commande et de calcul (15) est configurée de telle manière que les impulsions de pression générées par le rythme cardiaque du patient sont mesurées dans l'embranchement artériel et dans l'embranchement veineux (6, 7) du circuit extracorporel (I), dans lequel l'amplitude des impulsions de pression mesurées dans l'embranchement artériel est comparée à une première valeur limite et l'amplitude des impulsions de limite mesurées dans l'embranchement veineux est comparée à une deuxième valeur limite,
et que l'unité de commande et de calcul (15) est configurée de telle manière que le signal de commande est généré afin de démarrer la pompe à sang (9) quand l'amplitude des impulsions de pression mesurées dans l'embranchement artériel (6) est supérieure à la première valeur limite et quand l'amplitude des impulsions de pression mesurées dans l'embranchement veineux (7) est supérieure à la deuxième valeur limite.

9. Dispositif selon l'une quelconque des revendications 1 à 8, **caractérisé en ce que** le dispositif de surveillance présente un élément de temporisation (15A), et l'unité de commande et de calcul (15) est configurée de telle manière qu'une fois la pompe à sang (9) arrêtée, l'élément de temporisation (15A) est mis en marche et un signal de commande servant à fermer l'organe de fermeture veineux (17) est généré quand le laps de temps est écoulé.

10. Dispositif selon l'une quelconque des revendications 1 à 9, **caractérisé en ce que** la pompe à sang (9) est une pompe à sang d'occlusion.

11. Dispositif servant au traitement du sang extracorporel, qui présente un circuit sanguin extracorporel (I) avec un embranchement artériel (6) menant à une unité de traitement du sang (1) avec un raccordement artériel à un patient (5), et avec un embranchement veineux (7) partant de l'unité de traitement du sang (19), dans lequel un organe de fermeture veineux (17) est prévu en amont d'un raccordement veineux au patient (8), et une pompe à sang (9) servant à refouler le sang dans le circuit extracorporel (I), et une unité de commande et de calcul (15) centrale servant à commander la pompe à sang (9) et l'organe de blocage veineux (17),
**caractérisé en ce que** le dispositif de traitement du sang présente un dispositif servant à surveiller le circuit sanguin extracorporel selon l'une quelconque des revendications 1 à 10, dans lequel l'unité de commande et de calcul (15) centrale servant à commander la pompe à sang (9) et l'organe de blocage veineux (17) est configurée de telle manière qu'une fois le signal de commande du dispositif de surveillance servant à stopper la pompe à sang (9) reçu, la pompe à sang est stoppée et une fois le signal de commande du dispositif de surveillance servant à démarrer la pompe à sang reçu, la pompe à sang est démarrée, et une fois le signal de commande servant à fermer l'organe de fermeture veineux reçu, l'organe de fermeture veineux (17) est fermé.
